# EUROPEAN PATENT SPECIFICATION

(11) **EP 4 036 219 B1**
(45) Date of publication and mention of the grant of the patent: **08.11.2023**
(21) Application number: 21208495.8
(22) Date of filing: 16.11.2021
(51) Int. Cl.: C12N 1/20, A23K 10/18, A23L 3/3571, A23L 33/14, C12R 1/25

(54) **BACTERIAL STRAIN AND THE USE THEREOF**
BAKTERIENSTAMM UND SEINE VERWENDUNG
SOUCHE BACTÉRIENNE ET SON UTILISATION

(43) Date of publication of application: 03.08.2022
(73) Proprietor: Probitat Oy, 70211 Kuopio (FI)
(72) Inventor: Plumed-Ferrer, Carme, Kuopio (FI); Von Wright, Atte, Kuopio (FI)
(74) Representative: Berggren Oy

(56) References cited:
- EP-A1- 2 311 473
- GHEZIEL CHAHIRA ET AL: "Evaluating the Probiotic Potential of Strains from Algerian Infant Feces: Towards the Design of Probiotic Starter Cultures Tailored for Developing Countries", PROBIOTICS AND ANTIMICROBIAL PROTEINS, NEW YORK, NY ; HEIDELBERG : SPRINGER, NEW YORK, NY ; HEIDELBERG : SPRINGER, vol. 11, no. 1, 19 February 2018 (2018-02-19), pages 113-123, XP037245652, ISSN: 1867-1306, DOI: 10.1007/S12602-018-9396-9 [retrieved on 2018-02-19]
- GOOSSENS D. A. M. ET AL: "The effect of a probiotic drink with Lactobacillus plantarum 299v on the bacterial composition in faeces and mucosal biopsies of rectum and ascending colon", ALIMENTARY PHARMACOLOGY & THERAPEUTICS., vol. 23, no. 2, 1 January 2006 (2006-01-01), pages 255-263, XP55908156, GB ISSN: 0269-2813, DOI: 10.1111/j.1365-2036.2006.02749.x
- CEBECI AYSUN ET AL: "Properties of potential probiotic Lactobacillus plantarum strains", FOOD MICROBIOLOGY., vol. 20, no. 5, 1 October 2003 (2003-10-01), pages 511-518, XP55908087, GB ISSN: 0740-0020, DOI: 10.1016/S0740-0020(02)00174-0
- BOSCH M. ET AL: "Probiotic properties of Lactobacillus plantarum CECT 7315 and CECT 7316 isolated from faeces of healthy children : Lactobacillus plantarum strains as probiotics", LETTERS IN APPLIED MICROBIOLOGY, vol. 54, no. 3, 10 February 2012 (2012-02-10), pages 240-246, XP55908091, GB ISSN: 0266-8254, DOI: 10.1111/j.1472-765X.2011.03199.x
- ZHENG JINSHUI ET AL: "A taxonomic note on the genus Lactobacillus: Description of 23 novel genera, emended description of the genus Lactobacillus Beijerinck 1901, and union of Lactobacillaceae and Leuconostocaceae", INTERNATIONAL JOURNAL OF SYSTEMATIC AND EVOLUTIONARY MICROBIOLOGY, vol. 70, no. 4, 1 April 2020 (2020-04-01), pages 2782-2858, XP055831870, GB ISSN: 1466-5026, DOI: 10.1099/ijsem.0.004107 Retrieved from the Internet: URL:https://www.microbiologyresearch.org/d ocserver/fulltext/ijsem/70/4/2782_ijsem004 107.pdf?expires=1628778399&id=id&accname=s gid024200&checksum=393C98DAE8D2FFA7DAE5F8C EB968C536>

## Description

### Technical field

The present application relates to a bacterial strain and a biologically active culture thereof, as well as the uses of said bacterial strain and said biologically active culture in food- or feed-grade substance, food or feed composition and food or feed product.

### Background

It is well accepted in the art that probiotics are live micro-organisms contributing to the health and/or the well-being of a subject, such as a human or an animal, when being consumed. For example, it has been observed the influence of probiotics on intestine, wellbeing and balance of immune system of a subject. Also their effect on metabolism, including overcoming and prevention of diseases, protection of body against harmful microbiota and even providing positive effects on mental health has been suggested.

EP 2 311 473 A1 discloses *Lactobacillus plantarum* strains CECT 7527, 7528 and 7529, which have been isolated from human faeces. Gheziel Chahira et al. (Probiotics and antimicrobial proteins, vol. 11, no. 1, 19 February 2018, pp. 113-123) discloses *L. plantarum* strains derived from infant feces, for instance L. plantarum strain LSC3. Goossens D. A. M. et al. (Alimentary Pharmacology & Therapeutics, vol. 23, no. 2, 1 January 2006, pages 255-263) discloses *L*. *plantarum* strain 299v and a probiotic drink comprising the strain. Cebeci Aysun et al. (Food Microbiology, vol. 20, no. 5, 1 October 2003, pp. 511-518) discloses several *L. plantarum* strains, e.g. strains HU, NCIMB 1193, 80, DSM 20174. Bosch M. et al. (Letters in Applied Microbiology, vol. 54, no. 3, 10 February 2012, pp. 240-246) discloses the strains *L. plantarum* CECT 7314 and 7316, which have been isolated from human faeces.

However, probiotics are neither always effective with improvement in the gut microbiota, nor capable of contributing the quality of food or feed substance whereto they are added. In addition, in many cases a mixture of several probiotic strains is necessary for achieving an effect.

Thus, there is a continuous need for new probiotics.

### Summary

An aim of the invention is to provide a bacterial strain or a culture for probiotic use and other versatile uses, as well as to provide methods for using such strain or culture.

Thus, one aspect of the invention is a bacterial strain of *Lactiplantibacillus plantarum* Provege, deposited on 24 August 2021 with DSMZ under number DSM 33989. DSMZ stands for Deutsche Sammlung von Mikroorganismen und Zellkulturen GmbH, German Collection of Microorganisms and Cell Cultures GmbH.

Another aspect of the invention is a biologically active culture comprising the strain of *Lactiplantibacillus plantarum* Provege, deposited on 24 August 2021 with DSMZ under number DSM 33989. The biologically active culture comprises the strain and a cultural medium or residues of it.

Another aspect of the invention is an encapsulated product, such as freeze-dried powder, a tablet, or a capsule, comprising the strain of *Lactiplantibacillus plantarum* Provege, deposited on 24 August 2021 with DSMZ under number DSM 33989. The biologically active culture may be obtainable by incubating the strain in a cultural medium at 30 °C overnight, and preferably centrifuging the cultural medium to obtain a pellet and redissolving the pellet in another cultural medium or residues of it, for example peptone water containing 0.1% peptone.

Another aspect of the invention is a food or feed composition, a food or feed product, a formulation for food or feed enrichment, a food or feed supplement, or a nutraceutical formulation, comprising the strain, the biologically active culture, or the encapsulated product according to the presently disclosed invention.

Another aspect of the invention is a method of manufacturing a probiotic food or feed composition. Said method comprises preparing, and preferably also fermenting, a mixture of food or feed composition and the strain, the biologically active culture, or the encapsulated product according to the presently disclosed invention.

Another aspect of the invention is a method of increasing nutritional value of food- or feed-grade substance. Said method comprises fermenting a mixture of food- or feed-grade substance and the strain, the biologically active culture, or the encapsulated product according to the presently disclosed invention.

Another aspect of the invention is a method of prolonging shelf-life of a food or feed product. Said method comprises fermenting a mixture of a food or feed product or a starting material of a food or feed product and the strain, the biologically active culture, or the encapsulated product according to the presently disclosed invention.

Another aspect of the invention is use of the strain, the biologically active culture, or the encapsulated product as described herein, for example in manufacturing a food or feed composition, a food or feed product, a formulation for food or feed enrichment, a food or feed supplement, or a nutraceutical formulation.

The main embodiments are characterized in the independent claims. Various embodiments are disclosed in the dependent claims and the description. The features recited in dependent claims and in the embodiments are mutually freely combinable unless otherwise explicitly stated.

### Brief description of the drawings

Fig. 1 shows a microscopic view of L. plantarum Provege.
Fig. 2a - Fig. 2d show the effects of *Lactiplantibacillus plantarum Provege* in increasing abundance of gut bacteria *Fusicatenibacter*, *Tyzzerella, Oscillospira* and *Eubacterium,* respectively.
Fig. 3a - Fig. 3d show the effects of *Lactiplantibacillus plantarum Provege* in decreasing abundance of gut bacteria *Prevotella 2, Prevotella* 7, *Alloprevotella* and *Tyzzerella 4*, respectively.
Fig. 4 shows the levels of Enterobacteria and Lactic acid bacteria in gut before, during and after the consumption of smoothies containing *Lactiplantibacillus plantarum Provege.*
Fig. 5 shows the levels of Enterobacteria and Lactic acid bacteria in gut before, during and after the consumption of smoothies without *Lactiplantibacillus plantarum Provege.*
Fig. 6 shows the growth curves of mold on Prevege fermented breads and control samples.

### Deposits

The following strain deposition according to the Budapest Treaty on the International Recognition of Deposit of Microorganisms for the Purposes of Patent Procedure were made at the German Collection of Microorganisms and Cell Cultures GmbH, Mascherroder Weg I-Lb 38123 Braunschweig , Germany (DE) on 24 August, 2021: *Lactiplantibacillus plantarum* Provege assigned accession number DSM 33989. Said bacterial strain *Lactiplantibacillus plantarum* Provege is herein referred to as a bacterial strain according to the present invention, or *L. plantarum* Provege, or simply Provege. The strain was deposited by Probitat Oy, Kuopio, Finland. In the present specification, German Collection of Microorganisms and Cell Cultures GmbH is referred as DSMZ, which stands for its German name Deutsche Sammlung von Mikroorganismen und Zellkulturen GmbH.

### Detailed description

Unless being specified differently, the unit "vol.%", as used herein throughout the specification, refers to volume/volume percentage, or volume fraction expressed in percent, defined as the volume of a constituent divided by the volume of all constituents of the mixture prior to mixing, expressed in percent. The present invention relates to a new bacterial strain of *Lactiplantibacillus plantarum* Provege, isolated from the feces of humans. It has been surprisingly demonstrated that the strain has versatile benefits in various uses, such as for probiotic use, especially for improving digestibility and gut health of humans, for increasing nutritional value of food or feed-grade substance, and for prolonging self-life of food or feed products.

Thus, the present disclosure includes the presently disclosed bacterial strain *L. plantarum* Provege, as well as its mutant forms or derivatives which possess the same properties as the *L. plantarum* Provege. The *L. plantarum* Provege defined above is referred herein as the bacterial strain according to the present invention.

One aspect of the present disclosure provides a biologically active culture comprising the strain *L. plantarum* Provege or a mutant or derivative thereof.

The biological activity as used herein refers especially to viability and adhesion properties of the strain or the culture. Expression "viable" means that the cells are alive and viable in the body, such as in the intestine, of a subject, for at least the standard intestinal transit time (usually 12 to 48 hours). Viability is defined as the number of bacteria (cfu g⁻¹ ) that remain viable in their site of action to produce a beneficial health effect to the host. The number of bacteria may be calculated using conventional methods, e.g. based on microbe counts in a Petri dish. Expression "adhesion ability" means that the strain or culture is able to adhere to intestinal mucosa or to other desired target in the body of a subject. Good adherence prolongs the time that said strain can be active in the body of the subject.

The biologically active culture may be prepared from a frozen stock of the strain or from freeze-dried powder.

For preparing such a biologically active culture, *Lactiplantibacillus plantarum* Provege may be incubated at a cultural medium, for example a selective cultural medium, at 30 °C overnight, centrifuging the cultural medium to obtain a pellet and redissolving the pellet in another culture medium, such as a nonselective cultural medium. Types of culture media for microorganisms are well known to the skilled person in the art. For example, the strain may be inoculated using a 1 µl loop to scratch a frozen bacterial stock and adding it into 10 ml MRS broth (Lab M, Bury, Lancashire, UK) and incubated at 30 C overnight. The overnight broth is then centrifuged, the supernatant discarded, and the pellet redissolved in peptone water (0.1% peptone) or 0.9% sodium chloride. The biologically active culture containing the strain according to the present invention is thus ready for further use.

Freeze-dried powder of the strain according to the present invention may be prepared using conventional methods. Freeze drying bacteria is a multistep process which involves culturing the microbes, suspending them in a lyophilization medium/buffer, subjecting them to the freeze drying process, and then subsequently storing them properly.

Besides freeze drying bacteria, other stored forms of products of the bacterial strains according to the present invention may be prepared, such as capsules, tablets or similar products, which are collectively referred as encapsulated products.

Thus, the present disclosure provides an encapsulated product comprising the strain *L. plantarum* Provege or a mutant or derivative thereof. The encapsulated product may be, for example, freeze-dried powder, a tablet, a capsule, an effervescent tablet, or so on.

The term encapsulation refers to a process of entrapping a substance, i.e. the bacterial in the present application, into another substance, producing particles in the nanometer, micrometer or millimeter scale. A substance used to encapsulate is called coating membrane, shell, carrier or wall material, external phase or matrix. For specific encapsulation processes such as freeze-drying, the substances used to encapsulate are also called cryoprotectants. The wall material used in food products or processes should be food grade and must be able to form a barrier between the active agent and its surroundings. Encapsulation technologies used to keep to keep probiotic cell viable throughout storage, commercialization and use in food products, are known by the skilled person in the art.

### [probiotic use]

As mentioned, one object of the invention is to provide the bacteria strain as disclosed herein for probiotic use.

It is demonstrated that a food or feed composition comprising bacterial strains according to the present invention increases the level of many types of gut bacteria, whose existence has positive influence on some heath conditions, while decreases the level of many types of gut bacteria, whose existence has negative influence on certain conditions in a subject, such as a human or an animal, especially humans and pets. Thus, the bacterial strains according to the present invention contribute to the health condition of gut microbiota of a subject, such as a human or an animal, especially humans and pets. Accordingly, the present invention provides a method of manufacturing a probiotic food or feed composition.

Examples of above-mentioned gut bacteria having positive influence on some heath conditions include, but not limited to, *Fusicatenibacter*, *Tyzzerella, Oscillospira* and *Eubacterium.* By increasing the level of the gut bacterial is meant to increase the amount said gut bacterial from a naturally existing level in a subject. The Statistical significance is determined using the Students t-test.

Examples of above-mentioned gut bacteria having negative influence on some heath conditions include, but not limited to, *Prevotella 2*, *Prevotella 7*, *Alloprevotella* and *Tyzzerella 4.* By decreasing the level of the gut bacterial is meant to decrease the amount said gut bacterial from a naturally existing level in a subject. The Statistical significance is determined using the Students t-test.

A food or feed composition as disclosed herein refers a consumable composition comprising at least one food- or feed-grade substance. A food or feed-grade substance is a non-toxic and safe for consumption substance consumable to provide nutritional support for a subject, for example humans and animals. All ingredients comprised in a food or feed composition as disclosed herein are non-toxic and safe for consumption for the subject meant to consume such composition. Thus, a food- or feed-grades substance may be human food or animal feed, or in the synonymous expression such as food for humans, fodder for livestock or food/feed for pets. A food- or feed-grade substance may be plant, animal or fungal origin, containing nutrients, such as carbohydrates, fats, proteins, vitamins, and/or minerals. A food- or feed-grade substance according to the present invention includes but is not limited to a substance originally from dairy-, grain-, legumes-/pulses-, vegetable-, fruit-, berry-, fungus-, fish-, or meat- based material. The term "based material" is herein defined that the food or feed-grade substance is produced from a specific raw material such as dairy, grain, legumes/pulses, vegetable, fruit, berry, fungus, fish , or meat, and preferably the food or feed-grade substance contains at least 70% of the raw material(s). The food or feed-grade substance may be based on a mixture of different raw materials e.g. a mixture of dairy and grain or a mixture of meat and fruit. The food or feed-grade substance may be in its natural form or processed, such as fermented, baked, cooked, etc. A probiotic food or feed composition refers to a food or feed composition having probiotic properties. A probiotic food or feed composition according to the present invention comprises at least one food or feed-grade substance, and the bacterial strain according to the present invention, the biologically active culture, or the encapsulated product according to the present invention.

The present invention further provides a food or feed product, a formulation for food or feed enrichment, a food or feed supplement, or a nutraceutical formulation, comprising the strain according to the present invention, the biologically active culture, or the encapsulated product according to the present invention.

A food or feed product as disclosed herein refers to a product intended for being commercially available for consumers, and such product comprises the food or feed composition as defined. A food or feed product or a food or feed composition may be in the form of solid, semi-solid, gel or liquid. Examples of food or feed product include, but not limited to cheese, butter, bread, porridge, juice, smoothie, yogurt, milk, pudding, coffee, or the like.

Such food or feeds may be prepared in a manner known per se, e.g. by adding the the encapsulated product, biologically active culture or the bacterial strain according to the present invention to a suitable food or feed or food or feed-grade substance, in a suitable amount. For example, the bacterial strain according to the present invention, or the biologically active culture or the encapsulated product according to the present invention may be used in a manner known per se for the preparation of a fermented food or feed or food or feed-grade substance, e.g. in a manner known per se for the preparation of fermented food or feed products using the bacterial strain according to the present invention, the biologically active culture or the encapsulated product according to the present invention. In such methods, the bacterial strain according to the present invention, the biologically active culture or the encapsulated product according to the present invention may be used in addition to the micro-organism usually used, and/or may replace one or more or part of the micro-organism usually used. For example, in the preparation of fermented products such as smoothie, the bacterial strain according to the present invention, the biologically active culture or the encapsulated product according to the present invention may be added to or used as part of a starter culture or may be suitably added during such a fermentation.

Accordingly, there is provided according to the present invention a method of manufacturing a probiotic food or feed composition, comprising the steps:
- a) contacting the bacterial strain according to the present invention, the biologically active culture, or the encapsulated product according to the present invention with a food or feed composition;
- b) optionally, fermenting the food or feed composition obtained from the step a).

In one preferred embodiment, the method comprises the step b) fermenting the food or feed composition obtained from the step a)

Such food or feed composition having the probiotic properties may be made by e.g. adding the bacterial strains according to the present invention into a food or feed composition and proceed to fermentation. For example, the food or feed composition having the probiotic properties may be made by adding the biologically active culture as disclosed herein into food or feed or drink in a concentration of 0.01 - 10.00 vol.%. Unless being specified differently, the unit "vol.%", as used herein throughout the specification, refers to volume/volume percentage, or volume fraction expressed in percent, defined as the volume of a constituent divided by the volume of all constituents of the mixture prior to mixing, expressed in percent. Thus, in this case, the expression "adding the biologically active culture into food or feed or drink in a concentration of 0.01 - 10.00 vol.%" refers to the volume of the additive/constituent, i.e. the biologically active culture, in the volume of the whole composition, in this case food or feed composition, e.g. food or feed or drink, and the biologically active culture.

It is also possible to use freeze-dried sachets of bacterial strains according to the present invention for preparing such probiotic food or feed composition. The food or feed composition comprising the strain may then be fermented, thereby obtaining the probiotic food or feed composition, food or feed composition having the probiotic properties. It is noted that the concentration of bacterial strain added in to the food or feed composition may be tailored according to the applications. Likewise, the incubating/fermenting time may also be tailored according to the actual applications. For example, the inoculated food or feed may be incubated at 30 °C for 4-48 hours. It is found that a final dose of 10⁶ cfu of *Lactiplantibacillus plantarum* Provege is sufficient to provide probiotic effect.

In some examples, the probiotic food or feed composition contains 10⁷-10⁹ cfu/ml of *Lactiplantibacillus plantarum* Provege. In some preferred examples, a final dose of 10⁹ cfu of *Lactiplantibacillus plantarum* Provege may be recommended for consumers who would like to improved the gut conditions.

In some embodiments, the food or feed composition having the probiotic properties may be made by the steps of:
- adding the biologically active culture according to the present invention into a food or feed composition in a concentration of 0.01 - 10.00 vol.%, based on total volume of the whole composition having the active culture added in, preferably 0.05 - 5.00 vol.%, more preferably 0.10 - 1.00 vol.%, and mixing, thereby obtaining a mixture,
- optionally, incubating the mixture at 25 - 40 °C, preferably 30 - 35 °C, for 4-48 hours, preferably 6-24 hours.

To have the probiotic effect, the bacteria must remain viable in the product at least until it is consumed. Thus, incubating temperature shall not exceed 40 °C.

In some examples, the biologically active culture according to the present invention is added into the food or feed composition in a concentration of 0.10 - 1.00 vol.%, based on total volume of the whole composition having the active culture added in, and the incubating time is 6 - 24 hours at 30 °C, thereby obtaining the probiotic food or feed composition.

### [increasing nutritional value of food- or feed-grade substance ]

It is found that the bacteria strain according to the present invention contributes to the nutrient level of a food or feed-grade substance. Accordingly, the invention provides the bacteria strains as disclosed herein for increasing nutritional value of a food or feed-grade substance. Thus, there is provided a method of increasing nutritional value of a food or feed-grade substance, by fermenting a mixture of the food or feed-grade substance and the bacterial strain according to the present invention.

The meaning of food or feed-grade substance has been defined. Further examples of good-grade substance include, but not limited to, crops as food for humans, fodder for livestock or food/feed for pets, including grains or legumes/pulses, such as oat, quinoa or fava bean.

The phrase "increasing the nutritional value" refers to the amount of nutrients, including at least an amino acid and/or at least a mineral and/or at least a vitamin in the food or feed-grade substance, is increased from the naturally existing level in the unprocessed food or feed-grade substance. Furthermore, it is noted that the amount ofγ-aminobutyric acid (GABA), vitamin B12 and folic acid, may also be increased by the present method. It is to be noted that, although GABA is sometimes not classified as an amino acid in the art, here GABA is covered by the meaning of amino acid of the claimed invention.

According to some embodiments of the present method, at least the following nutrients may be simultaneously and significantly increased: Alanine, Arginine, Aspartic acid, Glutamic acid, Glycine, Histidine, Isoleucine, Leucine, Lysine, Methionine, Phenylalanine, Proline, Serine, Taurine, Threonine, Tyrosine, Valine, and GABA.

According to some embodiments of the present method, at least the following nutrients may be simultaneously and significantly increased: Alanine, Aspartic acid, Cysteine, Glutamic acid, Glycine, Histidine, Isoleucine, Leucine, Lysine, Methionine, Ornithine, Phenylalanine, Proline, Serine, Taurine, Threonine, Valine, and GABA.

According to some embodiments of the present method, at least the following nutrients may be simultaneously and significantly increased: calcium, iron, and zinc.

Such food or feed-grade substance having increased nutritional value may be made by e.g. adding the bacterial strains according to the present invention into a food or feed-grade substance and proceed to fermentation. For example, the food or feed-grade substance having increased nutritional value may be made by adding the biologically active culture as disclosed herein into a food or feed-grade substance, e.g. grans or legumes/pulses, in a concentration of 0.01 - 10.00 vol.%, based on total volume of the whole substance having the active culture added in.

It is noted that the amount of bacterial strain added in to the food or feed-grade substance may be tailored according to the applications. Likewise, the incubating/fermenting time may also be tailored according to the actual applications. For example, the inoculated food or feed may be incubated at 30 °C for 4 - 48 hours.

In some embodiments, the method of increasing nutritional value of a food or feed-grade substance may comprise
- adding the biologically active culture according to the present invention into a food or feed-grade substance in an amount of 0.01 - 10.00 vol.%, based on total volume of the whole substance having the active culture added in, preferably 0.05 - 5.00 vol.%, more preferably 0.10 - 1.00 vol.%, thereby obtaining a mixture;
- fermenting the mixture at 25 - 40 °C, preferably 30 - 35 °C, for 4 - 48 hours, preferably 12 - 24 hours;
- drying the fermented mixture at 40 - 120 °C, preferably 50 - 80 °C, more preferably 60 - 70 °C, for 4 - 96 hours, more preferably 24 - 48 hours.

The food or feed-grade substance may be pre-treated. For example, the food or feed-grade substance may be sterilized, e.g. by boiling, from endogenous bacteria.

In some embodiments, the biologically active culture according to the present invention is added into the food or feed-grade substance in a concentration of 0.10 - 1.00 vol.%, based on total volume of the whole substance having the active culture added in, and the incubating time is 12 - 24 hours at 30 °C, and the drying time is 24 - 48 hours at 65 °C.

### [prolonging shelf-life of food or feed products]

It is found that the bacteria strains according to the present invention contributes to the shelf-life of a food or feed product or a starting material of a food or feed product. It is found that the bacteria strains according to the present invention have antifungal activity. Accordingly, the invention provides the bacteria strains as disclosed herein for prolonging shelf-life of a food or feed product or a starting material of a food or feed product. Thus, there is provided a method prolonging shelf-life of a food or feed product, by fermenting a mixture of the food or feed product or a starting material of the food or feed product and the bacterial strains according to the present invention. The term "starting material of a food or feed product" refers to an ingredient or material that is used to manufacturing a food or feed product. A starting material may be an unprocessed raw material or processed material meant for further use in making the food or feed product. A starting material may be a food or feed-grade substance as defined herein. For example, grains or legumes/pulses, or flour or dough of grains or legumes/pulses, fall within the meaning of a starting material of a food or feed product which may be bread, baking and so on. It is to be noted that in this example flour as such falls within the meaning of a good product as well.

Is it found that when a food or feed product or a starting material of a food or feed product fermented with the bacterial strain according to the present invention or with the biologically active culture or the encapsulated product according to the present invention, the molding process of the food or feed product is a lot suppressed. For example, after contacting with a fungal source, the food or feed product fermented with the bacterial strain according to the present invention may start to grow mold at least one day later than those without the bacterial strain according to the present invention. Further, the growing speed of the mold of the food or feed product fermented with the bacterial strain according to the present invention may be as twice slower as those without the bacterial strain according to the present invention. Thus, by using the bacterial strain according to the present invention there is no need to add preservatives into the food or feed product.

Such food or feed product having prolonged shelf-life may be made by e.g. adding the bacterial strains according to the present invention into a product and proceed to fermentation. For example, the food or feed product substance having prolonged shelf-life may be made by adding the biologically active culture as disclosed herein into a food or feed product, e.g. flour, in a concentration of 0.01 - 10.00 vol.%, based on total volume of the whole product having the active culture added in.

It is noted that the amount of bacterial strain added in to the food or feed product may be tailored according to the applications. Likewise, the incubating/fermenting time may also be tailored according to the actual applications. For example, the inoculated food or feed may be incubated at 30 °C for 4 - 48 hours.

In some embodiments, the method of prolonging shelf-life of a food or feed product may comprise
- adding the biologically active culture according to the present invention into a food or feed product in an amount of 0.01 - 10.00 vol.%, based on total volume of the whole product having the active culture added in, preferably 0.05 - 5.00 vol.%, more preferably 0.10 -1.00 vol.%, thereby obtaining a mixture;
- fermenting the mixture at 25 - 40 °C, preferably 30 - 35 °C, for 4 - 48 hours, preferably 12 - 24 hours.

The food or feed product may be pre-treated. For example, the food or feed product may be sterilized, e.g. by boiling, from endogenous bacteria.

In some embodiments, the biologically active culture according to the present invention is added into the food or feed-grade substance in a concentration of 0.10 - 1.00 vol.%, based on total volume of the whole substance having the active culture added in, and the incubating time is 12 - 24 hours at 30 °C, and the drying time is 24 - 48 hours at 65 °C. Afterwards, the food or feed-grade substance is made into a food or feed product.

### Examples

The present invention is further described by the following examples which should not be construed as limiting the scope of the invention.

### Example 1: LACTIPLANTIBACILLUS PLANTARUM PROVEGE CULTIVATION AND IDENTIFICATION

### Sample preparation

10 g of human faeces were mixed with 90 ml of peptone water (0.1% peptone or 0.85% NaCl) and homogenized in a waring blender (Stomacher 400). This was the 10⁻¹ dilution.

### Sample dilution

Tenfold dilutions of each sample were prepared using peptone water.

### Selective media

De Mann, Rogosa and Sharpe (MRS) agar (Lab M, Bury, Lancashire, UK) was used to make agar plates. This media allows the growth of lactic acid bacteria.

### Inoculation

Bacterial numbers were determined by plating 0.1 ml of three appropriated dilutions, in duplicate, in MRS agar plates and incubated at 30 °C for 48 hours.

### Species selection

100 differently looking bacterial colonies were picked and re-inoculated in MRS agar plates and incubated for their purification.

### Species identification

API 50 CH strips and API 50 CHL medium (Bio Me'rieux, Marcy-l'Etoile, France) were used to identify lactic acid bacteria at the species level.

### Strain identification

Genomic DNA was extracted with Nucleo^{®}Tissue Kit (Macherey-Nagel, Düren, Germany) using Support protocol for bacteria.

The genetic diversity of the 100 isolates was analysed by Random Amplification of Polymorphic DNA (RAPD-PCR) with primers P2 (50-GAT CGG ACGG-30), P16 (50-TCG CCA GCC A-30) and P17 (50-CAG ACAAGC C-30). The RAPD fingerprints were, analysed using the software GELCOMPAR II (Applied Maths, Version 6.5, Sint-Martens-Latem, Belgium). RAPD dendrograms were obtained with hierarchical cluster analysis (UPGMA, Unweighted Pair Group Method with Arithmetic Mean). The similarity of band profiles was calculated based on the Pearson's correlation coefficient.

At least one representative of each RAPD cluster was chosen for identification by 16S rRNA gene sequencing. Primers 27f (50-AGA GTT TGA TCC TGG CTC AG-30) and 685r (50-TCT ACG CAT TTC ACC GCT AC-30) were used to obtain a fragment of approx. 650 bp. PCR products were purified with NucleoSpin^{®}Extract II kit (Macherey-Nagel) prior to sequencing (LGC Genomics GmbH, Berlin, Germany). The identification of isolates was obtained through the GenBank DNA database using the BLAST algorithm (http://www.ncbi.nlm.mnih.gov). Sequences showing at least 99% similarity levels are accepted.

### A Lactiplantibacillus plantarum strain was identified this way and named Lactiplantibacillu plantarum Provege (L. plantarum Provege)

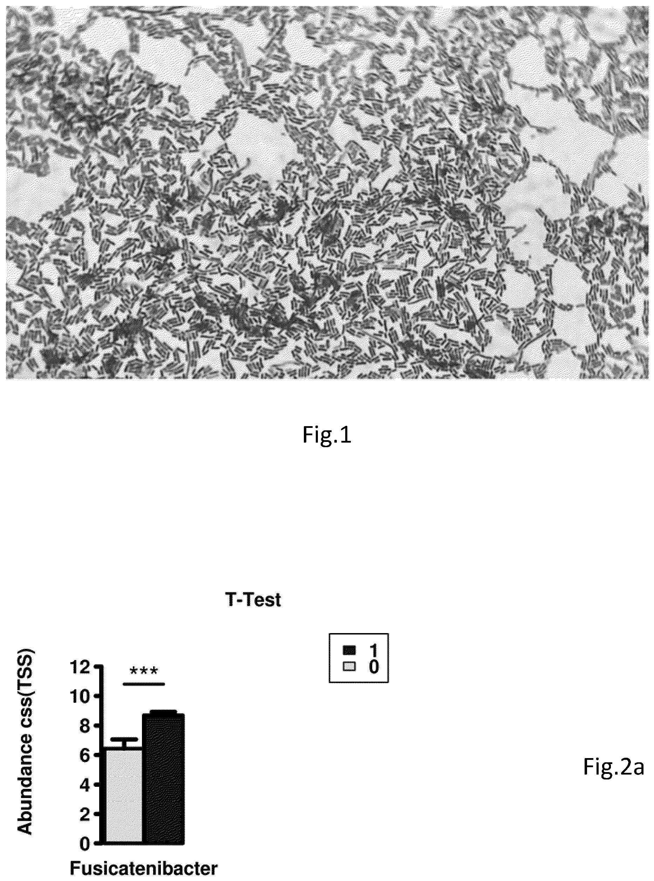

Fig. 1 shows a 100X microscopic view of *L. plantarum* Provege under an optical microscope.

### Example 2: GUT MICROBIOTA RESULTS - 1

### Method

Stool samples from all participants (control and probiotic groups) were obtained before (run-in), during and after (washout) the consumption of the quinoa smoothie, with and without 10⁹ cfu/ml of Lactiplantibacillus plantarum Provege.

Stool samples were kept frozen after collection at -20 °C until they were delivered to the laboratory and then stored at -80 °C until analysis.

DNA extraction was performed by using KingFisher and InviMag^{®} Stool DNA kit (Invitek GmbH, Berlin, Germany). The DNA was harvested using an automatic magnetic-particle purifying system, KingFisher (type: 700, Thermo Fischer Scientific Oy, Vantaa, Finland).

The V3-V4 region of 16S rDNA gene was amplified by PCR using Illumina adapter overhang nucleotide sequences following Illumina protocols. After 16S rDNA gene amplification, the mutiplexing step was performed using Nextera XT Index Kit. 1µl of the PCR product was checked with a Bioanalyzer DNA 1000 chip and libraries were sequenced using a 2×300pb paired-end run (MiSeq Reagent kit v3) on a MiSeq-Illumina platform (FISABIO sequencing service, Valencia, Spain).

The quality-filtered sequences were checked for chimera, and the nonchimeric sequences were processed using a QIIME pipeline (version 1.9.0). The sequences were clustered at 97% of identity into operational taxonomic units (OTUs), and representative sequences were taxonomically classified based on the Greengenes 16S rRNA gene database (version 13.8).

The microbial differences between the two developmental stages and the two treatment groups were analyzed using Calypso version 8.56 with data normalized by cumulative sum scaling, which corrects the bias in the assessment of differential abundance introduced by total sum normalization.

For the multivariate analysis of bacterial genera, a discriminant analysis of principal components (DAPC) was run considering groups based on developmental stage and GBH treatment. An analysis of variance (ANOVA) test was used to analyze the differences between the relative abundances of microbial groups in the Colorado potato beetle and between the Shannon diversity index, species richness and Chao1 index.

### Results

Hereunder shows the results of samples taken during the consumption of the quinoa smoothie.

Fig. 2a - Fig. 2d show the effects of *Lactiplantibacillus plantarum Provege,* i.e. increased abundance of gut bacteria *Fusicatenibacter*, *Tyzzerella*, *Oscillospira* and *Eubacterium,* respectively. Statistical significance was calculated using the Students t- test. The grey bar, denoted 0, is the result from the control group, where the participants consumed the quinoa smoothie without *Lactiplantibacillus plantarum* Provege.The black bar, denoted 1, is the result from the probiotic group, where the participants consumed the quinoa smoothie containing 10⁹ cfu/ml of *Lactiplantibacillus plantarum* Provege.

In patients with ulcerative colitis there was a significant decrease in the genus *Fusicatenibacter.* We found an increase of *Fusicatenibacter* in the probiotic group when consuming the probiotic strain as shown in Fig. 2a. This might indicate that consuming this strain has a protective effect against ulcerative colitis.

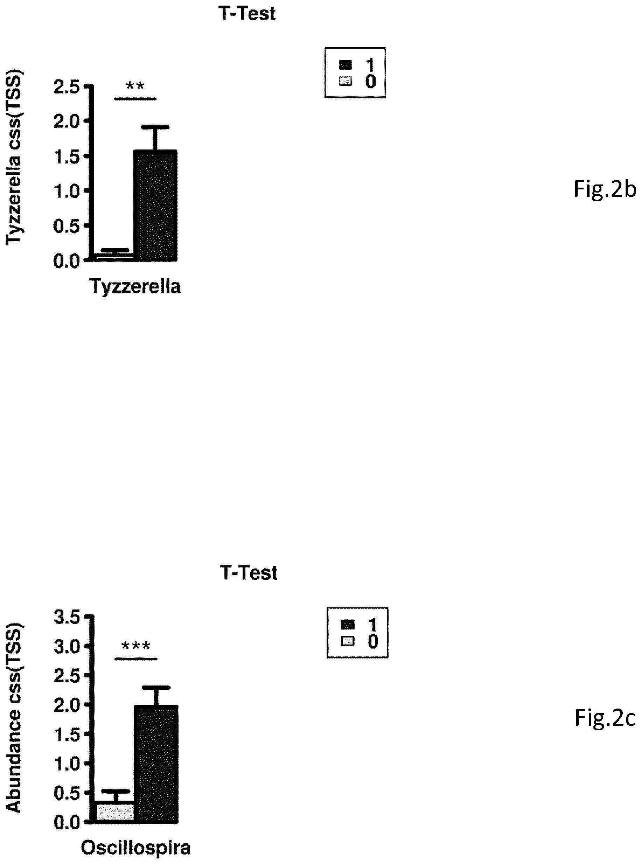

It has also been associated a decrease in *Tyzzerella* and *Fusicatenibacter* in patients with esophageal cancer and therefore, an increase of these bacteria in the probiotic consuming group might result in a protective effect. We found an increase of *Tyzzerella* in the probiotic group when consuming the probiotic strain as shown in Fig. 2b.

Recently, several human gut microbiota investigations have detected *Oscillospira* and demonstrated its underlying significance for host health. *Oscillospira* was reported to be less abundant in patients with inflammatory bowel disease and pediatric nonalcoholic steatohepatitis.

We found that *Oscillospira* is closely related to human health because its abundance was positively correlated with microbial diversity, high density lipoprotein, and sleep time, and was inversely correlated with diastolic blood pressure, systolic blood pressure, fasting blood glucose, triglyceride, uric acid and Bristol stool type. Moreover, random forest analysis with five-fold cross validation showed *Oscillospira* could be a predictor of low BMI and constipation in the subset. We found an increase of *Oscillospira* in the probiotic group when consuming the probiotic strain as shown in Fig. 2c.

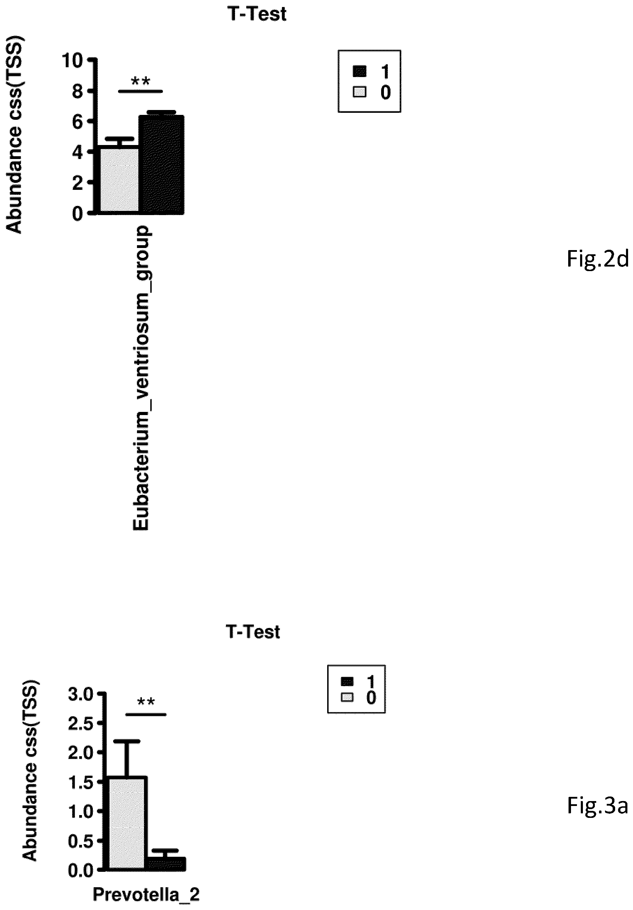

The genus consists of phylogenetically, and quite frequently phenotypically, diverse species, making *Eubacterium* a taxonomically unique and challenging genus. Several members of the genus produce butyrate, which plays a critical role in energy homeostasis, colonic motility, immunomodulation and suppression of inflammation in the gut. *Eubacterium* spp. also carry out bile acid and cholesterol transformations in the gut, thereby contributing to their homeostasis. Gut dysbiosis and a consequently modified representation of *Eubacterium* spp. in the gut, have been linked with various human disease states. We found an increase of *Eubacterium* in the probiotic group when consuming the probiotic strain as shown in Fig. 2d.

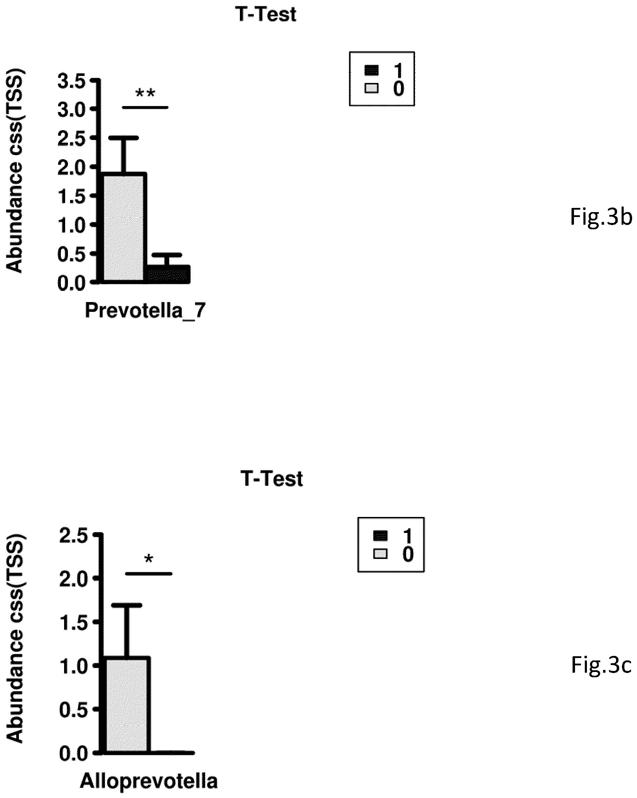

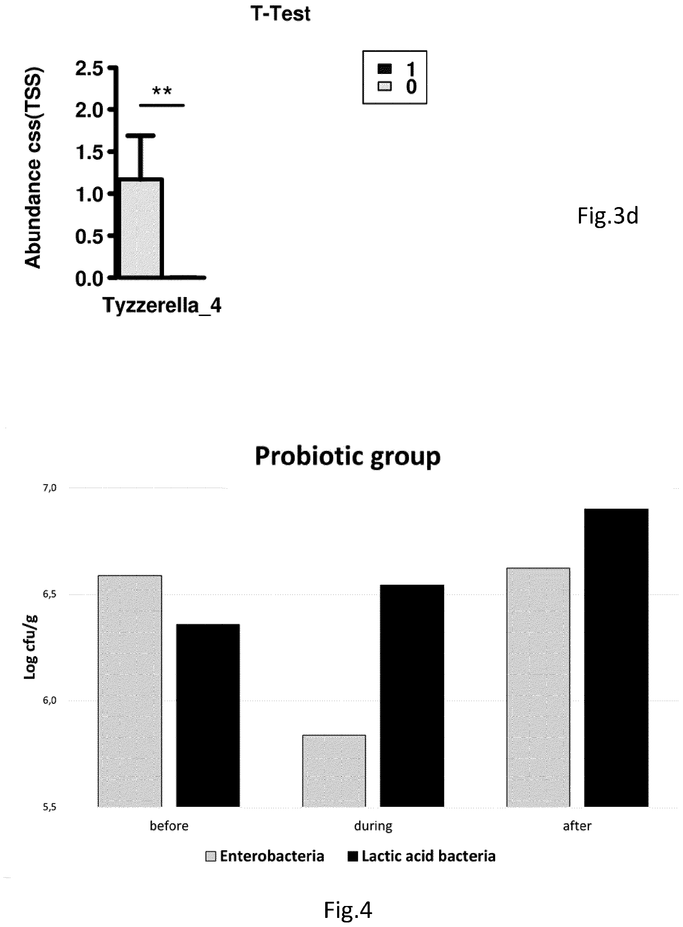

Fig. 3a - Fig. 3d show the effects of *Lactiplantibacillus plantarum Provege* in decreasing abundance of gut bacteria *Prevotella 2*, *Prevotella* 7, *Alloprevotella* and *Tyzzerella 4*, respectively. Statistical significance was calculated using the Students t- test. The grey bar, denoted 0, is the result from the control group, where the participants consumed the quinoa smoothie without *Lactiplantibacillus plantarum* Provege.The black bar, denoted 1, is the result from the probiotic group, where the participants consumed the quinoa smoothie containing 10⁹ cfu/ml of *Lactiplantibacillus plantarum* Provege.

It has been suggested that *Prevotella* is a beneficial bacteria as it is associated with a plant-rich diet, however, high levels of *Prevotella* have also been linked to obesity and significantly associated with BMI, insulin resistance in nondiabetic people, hypertension and non-alcoholic fatty liver disease. Furthermore, *Prevotella* has also been linked to high blood pressure and impaired glucose metabolism. *Prevotella 2*, *Prevotella 7 Alloprevotella* and *Tyzzerella* 4 have also been associated with cardiovascular disease (CVD) risk.

Thus, a reduction in the abundance of *Prevotella 2*, *Prevotella 7 Alloprevotella* and *Tyzzerella* 4 in the group consuming *L. plantarum Provege* might suggest an improvement in their gut microbiota, as shown in Fig. 3a to Fig. 3d.

### Example 3: GUT MICROBIOTA RESULTS - 2

### GROUP RESULTS (all participants)

During the quinoa smoothie intervention, the balance gut microbiota was studied. The intervention consisted in two groups. The first group consumed a **probiotic quinoa smoothie** and the second, a **control quinoa smoothie.** The only difference between the two groups was the added probiotic bacteria. The levels of bacteria in the probiotic smoothie were10⁹ cfu/ml.

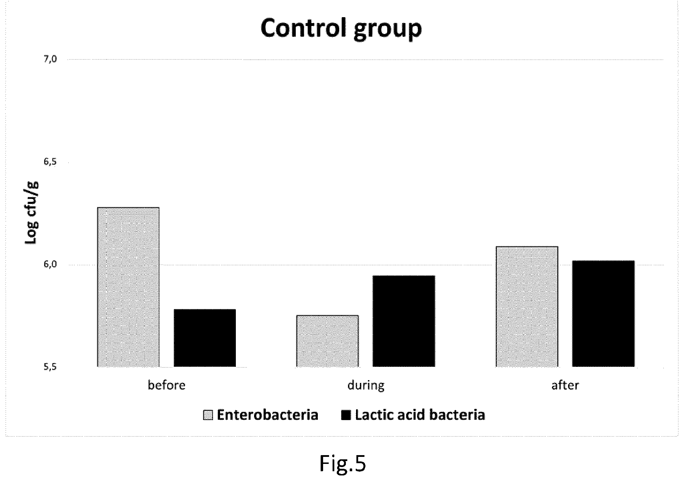

Figure 4 and 5 show the gut microbial balance results as the mean of **all participants** in each group. Figure 4 shows the results of the participants consuming the probiotic smoothie and figure 5 the participants consuming the control smoothie. The results are shown in 3 stages:
- Before consumption (visit 1 and 2)
- During the consumption (visit 3 and 4)
- After the consumption (visit 5)

This study was done using culture dependent method based on growing and selecting microbes and focused on Enterobacteria, known to have negative influence on gut microbiota, and Lactic acid bacteria, known to have positive influence on gut microbiota.

### The results for the probiotic consuming group showed:

### Before consuming:

Enterobacteria are in higher amounts than Lactic acid bacteria, which indicates a normal state of gut microbiota.

### During the consumption:

The amount of Enterobacteria are reduced while the amount of Lactic acid bacteria are increased.

### After the consumption:

Lactic acid bacteria stays in high amounts.

The results suggest that the beneficial effect of consuming the probiotic bacteria was still present at least two weeks after stopping their consumption. Thus, consuming the probiotic stimulates beneficial bacteria and this stimulation is present even after the probiotic is not present anymore.

**Fig 4****.** shows the levels of Lactic acid bacteria and Enterobacteria before consuming the probiotic smoothie, during one month of consumption and two weeks after stopping the consumption of the smoothie. The groups sharing the superscript letter (within same bacteria) are not significantly different (p<0.05)

### The results for the control group showed:

### Before consuming:

Enterobacteria are in higher amounts than Lactic acid bacteria, which indicates a normal state of gut microbiota.

### During the consumption:

The amount of Enterobacteria is reduced while the amount of Lactic acid bacteria increases.

These results are not statistically significant. This means that there is a tendency to reduce Enterobacteria and increase Lactic acid bacteria but the difference is very small.

### After the consumption:

Lactic acid bacteria are still in high amounts.

This result is also not statistically significant, so Lactic acid bacteria stayed higher than in the original levels but the difference is very small.

**Fig 5****.** shows the levels of Lactic acid bacteria and Enterobacteria before consuming the control smoothie, during one month of consumption and two weeks after stopping the consumption of the smoothie. No statistical significant differences were observed.

**CONCLUSION:** The consumption of the probiotic quinoa smoothie containing *Lactiplantibacillus plantarum Provege* can stimulate intestinal good bacteria and protect a subject from any intestinal dysregulation. This stimulation is still present even after the probiotic is not present anymore. From the control group, we can conclude that even without the probiotic, the gut microbiota has a tendency to reduce the Enterobacteria and increase the Lactic acid bacteria.

This effect is most likely due to the quinoa, which contains fiber (prebiotic). The prebiotic is the "food" that Lactic acid bacteria needs to be also stimulated.

When the Lactic acid bacteria of gut microbiota is stimulated (higher amounts than normal), the gut is more protected to different disturbances (stress, infections, etc.).

### Example 4: LACTIPLANTIBACILLUS PLANTARUM PROVEGE USED TO INCREASE THE NUTRITIONAL VALUE

### Increased nutritional value of grains and legumes/pulses:

Grains or legumes/pulses were boiled to sterilize them from any endogenous bacteria. Wet grains or legumes/pulses were inoculated with 1 % of Lactiplantibacillus plantarum Provege and incubated at 30 °C for 12 - 24 hours. After the fermentation step, they were dried at 65 °C for 24 - 48 hours. Once dried, they were milled into flour.

Flours were analyzed at Eurofins Scientific Finland.

### Increased in free amino acids:

| **QUINOA** | g/100g | | **FAVA BEAN** | g/100g | |
|---|---|---|---|---|---|
| | **NFQ** | **FQ** | | **NFFB** | **FFB** |
| **Alanine** | 0.018 | 0.253 | **Alanine** | 0.014 | 0.354 |
| Arginine | 0.071 | 0.085 | Arginine | 0.584 | 0.15 |
| Aspartic acid | 0.028 | 0.292 | Aspartic acid | 0.06 | 0.274 |
| Cysteine | <0.007 | <0.007 | Cysteine | <0.007 | 0.014 |
| Glutamic acid | 0.049 | 0.297 | Glutamic acid | 0.089 | 0.505 |
| Glycine | <0.01 | 0.98 | Glycine | <0.01 | 0.072 |
| **Histidine** | <0.018 | 0.032 | **Histidine** | <0.018 | 0.043 |
| **Isoleucine** | <0.018 | 0.109 | **Isoleucine** | <0.018 | 0.175 |
| **Leucine** | <0.008 | 0.19 | **Leucine** | <0.008 | 0.311 |
| **Lysine** | <0.007 | 0.064 | **Lysine** | 0.01 | 0.105 |
| **Methionine** | <0.008 | 0.043 | **Methionine** | <0.008 | 0.018 |
| Ornithine | <0.01 | <0.01 | Ornithine | <0.01 | 0.052 |
| Phenylalanine | <0.016 | 0.104 | Phenylalanine | <0.016 | 0.179 |
| Proline | <0.01 | 0.195 | Proline | 0.021 | 0.199 |
| Serine | <0.008 | 0.099 | Serine | <0.008 | 0.083 |
| Taurine | <0.009 | 0.96 | Taurine | <0.009 | 0.248 |
| **Threonine** | <0.003 | 0.08 | **Threonine** | 0.003 | 0.056 |
| **Tyrosine** | <0.012 | 0.079 | **Tyrosine** | 0.012 | <0.012 |
| **Valine** | 0.008 | 0.133 | **Valine** | <0.008 | 0.197 |
| Tryptophan free | 0.012 | 0.021 | Tryptophan free | <0.01 | 0.022 |
| GABA (g/kg) | 0.07 | **0.988** | GABA (g/kg) | <0.05 | 0.434 |

### Increased in minerals:

| **QUINOA** | mg/kg | | **FAVA BEAN** | mg/kg | |
|---|---|---|---|---|---|
| | **NFQ** | **FQ** | | **NFFB** | **FFB** |
| Calcium | 520 | 730 | Calcium | 1200 | 1400 |
| Iron | 39 | 44 | Iron | 49 | 58 |
| Zinc | 27 | 32 | Zinc | 47 | 54 |

| | | | | | |
|---|---|---|---|---|---|
| NFQ: non-fermented quinoa FQ: fermented quinoa NFFB: non-fermented fava bean FFB: fermented fava bean | | | | | |

### Example 5: LACTIPLANTIBACILLUS PLANTARUM PROVEGE USED TO INCREASE SHELF-LIFE

### 1. Antifungal activity in agar plates:

### Method:

LAB were inoculated on MRS agar plates as two-centimeter-long lines. After 24 hours of incubation at 30 °C the plates were overlaid with 10ml LB soft agar (2.5% LB, 0.7% agar) which was inoculated with 0.1% molds (Apergillus oryzae 1011 or Aspergullus oryzae 66222). The plates were then incubated at 30 °C for 48 hours. Clear zones of inhibition around the bacteria lines on the plates were examined and the areas of the clear zones were measured.

### Results:

| | **Aspergillus oryzae 1011** | | **Aspergillus oryzae 66222** |
|---|---|---|---|
| | Diameter (cm) | | Diameter (cm) |
| ***L. plantarum Provege*** | **0.79** | ***L. plantarum Provege*** | **0.93** |
| ***L. casei*** | 0.51 | ***L. casei*** | 0.69 |
| ***L. crustorum*** | 0.13 | ***L. crustorum*** | 0.22 |
| ***Ln. citreum*** | 0.18 | ***Ln. citreum*** | 0 |
| ***L. plantarum*** | 0.53 | ***L. plantarum*** | 0.74 |

As the results show, plates with *L. plantarum Provege* had the largest clear zones as compared with other strains tested, suggesting the antifungal activity of *L. plantarum Provege.*

### 2. Antifungal activity in L. plantarum Provege fermented quinoa bread

Dark bread and white bread were prepared according to a recipe commonly known in the industry, using flours with and without Provege fermentation obtained according to Example 4.

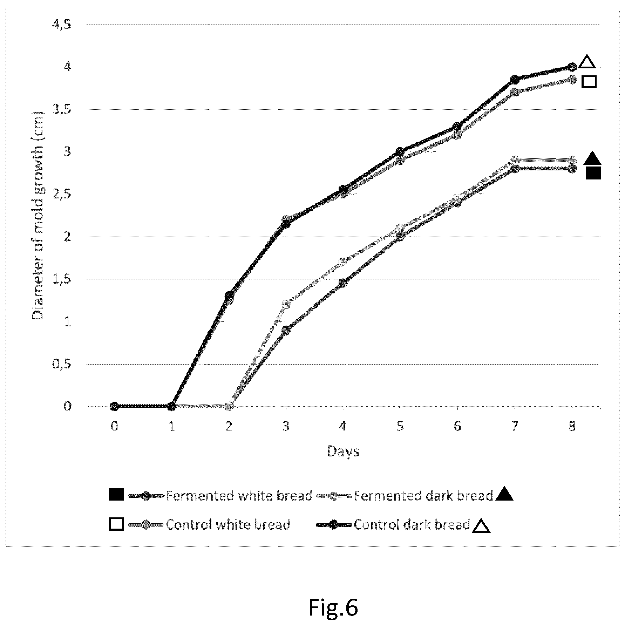

The sliced breads were studied according to their ability to resist fungal growth. Duplicates of each sample were made. The slices were inoculated with 50µl *Aspergillus oryzae* suspension (a loop of mold diluted in 1 ml of peptone water). The plates were covered and stored in plastic bags. Samples were incubated at room temperature. Samples without inoculation were as controls and stored in the same way. The growth of the mold was examined every day during eight days by observing the appearance and measuring the growth diameter, as shown in Fig.6. As can be seen in Fig. 7, the Provege fermented breads (indicated with symbols ■,▲) A) started to grow mold later and slower than the control samples (indicated with symbols □, △).

## Claims

1. A bacterial strain of *Lactiplantibacillus plantarum* Provege, deposited on 24 August, 2021 with DSMZ under number DSM 33989.

2. A biologically active culture comprising the strain according to claim 1, and a cultural medium or residues of it, for example peptone water containing 0.1% peptone.

3. An encapsulated product, such as freeze-dried powder, a tablet, or a capsule, comprising the strain according to claim 1.

4. A food or feed composition, a food or feed product, a formulation for food or feed enrichment, a food or feed supplement, or a nutraceutical formulation, comprising the strain according to claim 1, the biologically active culture according to claim 2, or the encapsulated product according to claim 3.

5. A method of manufacturing a probiotic food or feed composition, comprising the steps:
- a) contacting the bacterial strain according to claim 1, the biologically active culture according to claim 2, or the encapsulated product according to claim 3, with a food or feed composition;
optionally ,
- b) fermenting the food or feed composition obtained from the step a).

6. The method according to claim 5, wherein
- the step a) comprises adding the biologically active culture according to claim 2 into the food or feed composition in a concentration of 0.01 - 10.00 vol.%, based on total volume of the whole composition having the active culture added in, preferably 0.05 - 5.00 vol.%, more preferably 0.10 - 1.00 vol.%, and mixing, thereby obtaining a mixture; and
- the step b) comprises incubating the mixture at 25 - 40 °C, preferably 30 - 35 °C, for 4 - 48 hours, preferably 6 - 24 hours.

7. A method of increasing nutritional value of food- or feed-grade substance, comprising:
- fermenting a mixture of a food- or feed-grade substance and the bacterial strain according to claim 1, the biologically active culture according to claim 2, or the encapsulated product according to claim 3.

8. The method according to claim 7, wherein the nutritional value includes the quantity value of at least an amino acid and/or at least a mineral and/or at least a vitamin, and
- the mixture comprises the biologically active culture according to claim 2 in a concentration of 0.01 - 10.00 vol.%, based on total volume of the mixture, preferably 0.05 - 5.00 vol.%, more preferably 0.10 - 1.00 vol.% in the food- or feed-grade substance; and
- fermenting the mixture is performed at 25 - 40 °C, preferably 30 - 35 °C, for 4 - 48 hours, preferably 12 - 24 hours; and the method optionally further comprises
- drying the fermented mixture at 40 - 120 °C, preferably 50 - 80 °C, more preferably 60 - 70 °C, for 4 - 96 hours, more preferably 24 - 48 hours.

9. The method according to claim 7 or 8, wherein the food- or feed-grade substance is grains or legumes/pulses, such as oat, quinoa or fava bean.

10. The method according to any of claims 7 to 9, wherein the amino acid is alanine, histidine, isoleucine, leucine, lysine, methionine, threonine, tyrosine, or valine.

11. The method according to any of claims 7 to 10, wherein the mineral is calcium, iron, or zinc.

12. A method of prolonging shelf-life of a food or feed product, comprising fermenting a mixture of a food or feed product or a starting material of a food or feed product and the bacterial strain according to claim 1, the biologically active culture according to claim 2, or the encapsulated product according to claim 3.

13. The method according to claim 12, wherein
- the mixture comprises the biologically active culture according to claim 2 in a concentration of 0.01 - 10.00 vol.%, based on total volume of the mixture, preferably 0.05 - 5.00 vol.%, more preferably 0.10 - 1.00 vol.%, and
- fermenting the mixture is performed at 25 - 40 °C, preferably 30 - 35 °C, for 4 - 48 hours, preferably 12 - 24 hours.

14. Use of the strain according to claim 1, the biologically active culture according to claim 2, or the encapsulated product according to claim 3, in manufacturing a food or feed composition, a food or feed product, a formulation for food or feed enrichment, a food or feed supplement, or a nutraceutical formulation.

## Patentansprüche

1. Bakterienstamm von *Lactiplantibacillus plantarum* Provege, hinterlegt bei der DSMZ am 24. August 2021 unter der Nummer DSM 33989.

2. Biologisch aktive Kultur, umfassend den Stamm nach Anspruch 1, und ein Kulturmedium oder Reste davon, z. B. Peptonwasser mit 0,1 % Pepton.

3. Eingekapseltes Produkt, wie z. B. gefriergetrocknetes Pulver, eine Tablette oder eine Kapsel, umfassend den Stamm nach Anspruch 1.

4. Lebensmittel- oder Futtermittelzusammensetzung, ein Lebensmittel- oder Futtermittelprodukt, eine Formulierung zur Anreicherung von Lebens- oder Futtermitteln, ein Lebensmittel- oder Futtermittelzusatz oder eine nutrazeutische Formulierung, welche den Stamm nach Anspruch 1, die biologisch aktive Kultur nach Anspruch 2 oder das eingekapselte Produkt nach Anspruch 3 enthält.

5. Verfahren zur Herstellung einer probiotischen Lebensmittel- oder Futtermittelzusammensetzung, umfassend die Schritte:
a) Inkontaktbringen des Bakterienstamms nach Anspruch 1, der biologisch aktiven Kultur nach Anspruch 2 oder des eingekapselten Produkts nach Anspruch 3 mit einer Lebensmittel- oder Futtermittelzusammensetzung; gegebenenfalls,
b) Fermentieren der aus Schritt a) erhaltenen Lebensmittel- oder Futtermittelzusammensetzung.

6. Verfahren nach Anspruch 5, wobei
- der Schritt a) die Zugabe der biologisch aktiven Kultur nach Anspruch 2 zu der Lebensmittel- oder Futtermittelzusammensetzung in einer Konzentration von 0,01 - 10,00 Vol.-%, basierend auf dem Gesamtvolumen der gesamten Zusammensetzung, der die aktive Kultur zugesetzt wurde, vorzugsweise 0,05 - 5,00 Vol.-%, noch bevorzugter 0,10 - 1,00 Vol.-%, und Mischen umfasst, wodurch ein Gemisch erhalten wird; und
- der Schritt b) das Inkubieren des Gemisches bei 25 - 40 °C, vorzugsweise 30 - 35 °C, für 4 - 48 Stunden, vorzugsweise für 6 - 24 Stunden, umfasst.

7. Verfahren zur Erhöhung des Nährwerts einer Substanz in Lebensmittel- oder Futtermittelqualität, umfassend:
- Fermentieren eines Gemisches aus einer Substanz in Lebensmittel- oder Futtermittelqualität und dem Bakterienstamm nach Anspruch 1, der biologisch aktiven Kultur nach Anspruch 2 oder dem eingekapselten Produkt nach Anspruch 3.

8. Verfahren nach Anspruch 7, wobei der Nährwert den Mengenwert von mindestens einer Aminosäure und/oder mindestens eines Minerals und/oder mindestens eines Vitamins umfasst, und
- das Gemisch die biologisch aktive Kultur nach Anspruch 2 in einer Konzentration von 0,01 - 10,00 Vol.-% basierend auf dem Gesamtvolumen des Gemisches, vorzugsweise 0,05 - 5,00 Vol.-%, noch bevorzugter 0,10 - 1,00 Vol.-% in der Substanz in Lebensmittel- bzw. Futtermittelqualität umfasst; und
- das Fermentieren des Gemisches bei 25 - 40 °C, vorzugsweise 30 - 35 °C für 4 - 48 Stunden, vorzugsweise für 12 - 24 Stunden durchgeführt wird; und das Verfahren wahlweise ferner umfasst:
- Trocknen des fermentierten Gemisches bei 40 - 120 °C, vorzugsweise 50 - 80 °C, noch bevorzugter 60 - 70 °C für 4 - 96 Stunden, noch bevorzugter für 24 - 48 Stunden.

9. Verfahren nach Anspruch 7 oder 8, wobei es sich bei der Substanz in Lebensmittel- bzw. Futtermittelqualität um Getreide oder Hülsenfrüchte/Leguminosen wie z. B. Hafer, Quinoa oder Ackerbohnen handelt.

10. Verfahren nach einem der Ansprüche 7 bis 9, wobei es sich bei der Aminosäure um Alanin, Histidin, Isoleucin, Leucin, Lysin, Methionin, Threonin, Tyrosin oder Valin handelt.

11. Verfahren nach einem der Ansprüche 7 bis 10, wobei es sich bei dem Mineral um Kalzium, Eisen oder Zink handelt.

12. Verfahren zur Verlängerung der Haltbarkeitsdauer eines Lebensmittel- oder Futtermittelprodukts, umfassend das Fermentieren eines Gemisches aus einem Lebensmittel- oder Futtermittelprodukt oder einem Ausgangsmaterial eines Lebensmittel- oder Futtermittelprodukts und dem Bakterienstamm nach Anspruch 1, der biologisch aktiven Kultur nach Anspruch 2 oder dem eingekapselten Produkt nach Anspruch 3.

13. Verfahren nach Anspruch 12, wobei
- das Gemisch die biologisch aktive Kultur nach Anspruch 2 in einer Konzentration von 0,01 - 10,00 Vol.-%, basierend auf dem Gesamt-volumen des Gemisches, vorzugsweise 0,05 - 5,00 Vol.-%, noch bevorzugter 0,10 - 1,00 Vol.-% umfasst, und
- das Fermentieren des Gemisches bei 25 - 40 °C, vorzugsweise 30 - 35 °C, für 4 - 48 Stunden, vorzugsweise für 12 - 24 Stunden durchgeführt wird.

14. Verwendung des Stammes nach Anspruch 1, der biologisch aktiven Kultur nach Anspruch 2 oder des eingekapselten Produkts nach Anspruch 3 bei der Herstellung einer Lebensmittel- oder Futtermittelzusammensetzung, eines Lebensmittel- oder Futtermittelprodukts, einer Formulierung zur Anreicherung von Lebens- oder Futtermitteln, eines Lebensmittel- oder Futtermittelzusatzes oder einer nutrazeutischen Formulierung.

## Revendications

1. Souche bactérienne de *Lactiplantibacillus plantarum* Provege, déposée le 24 août 2021 auprès du DSMZ sous le numéro DSM 33989.

2. Culture biologiquement active comprenant la souche selon la revendication 1, et un milieu de culture ou des résidus de celui-ci, par exemple de l'eau peptonée contenant 0,1 % de peptone.

3. Produit encapsulé, tel qu'une poudre lyophilisée, un comprimé, ou une capsule, comprenant la souche selon la revendication 1.

4. Composition destinée à l'alimentation humaine ou animale, produit destiné à l'alimentation humaine ou animale, formulation pour l'enrichissement de l'alimentation humaine ou animale, complément dans l'alimentation humaine ou animale, ou formulation nutraceutique, comprenant la souche selon la revendication 1, la culture biologiquement active selon la revendication 2, ou le produit encapsulé selon la revendication 3.

5. Procédé de fabrication d'une composition probiotique destinée à l'alimentation humaine ou animale, comprenant les étapes de :
- a) mise en contact de la souche bactérienne selon la revendication 1, de la culture biologiquement active selon la revendication 2, ou du produit encapsulé selon la revendication 3, avec une composition destinée à l'alimentation humaine ou animale ;
facultativement,
- b) fermentation de la composition destinée à l'alimentation humaine ou animale obtenue à l'étape a).

6. Procédé selon la revendication 5, dans lequel
- l'étape a) comprend l'ajout de la culture biologiquement active selon la revendication 2 dans la composition destinée à l'alimentation humaine ou animale à une concentration de 0,01 à 10,00 % vol., rapporté au volume total de la composition entière dans laquelle a été ajoutée la culture active, de préférence 0,05 à 5,00 % vol., plus préférentiellement 0,10 à 1,00 % vol., et une homogénéisation, ce qui permet d'obtenir un mélange ; et
- l'étape b) comprend l'incubation du mélange à 25-40 °C, de préférence 30-35 °C, pendant 4 à 48 heures, de préférence 6 à 24 heures.

7. Procédé pour augmenter la valeur nutritive d'une substance de qualité alimentaire pour l'alimentation humaine ou animale, comprenant :
- la fermentation d'un mélange d'une substance de qualité alimentaire pour l'alimentation humaine ou animale et de la souche bactérienne selon la revendication 1, de la culture biologiquement active selon la revendication 2, ou du produit encapsulé selon la revendication 3.

8. Procédé selon la revendication 7, dans lequel la valeur nutritive inclut la valeur quantitative d'au moins un acide aminé et/ou d'au moins un minéral et/ou d'au moins une vitamine, et
- le mélange comprend la culture biologiquement active selon la revendication 2 à une concentration de 0,01 à 10,00 % vol., rapporté au volume total du mélange, de préférence 0,05 à 5,00 % vol., plus préférentiellement 0,10 à 1,00 % vol. dans la substance de qualité alimentaire pour l'alimentation humaine ou animale ; et
- la fermentation du mélange est effectuée à 25-40 °C, de préférence 30-35 °C, pendant 4 à 48 heures, de préférence 12 à 24 heures ; et le procédé comprend en outre facultativement
- le séchage du mélange fermenté, à 40-120 °C, de préférence 50-80 °C, plus préférentiellement 60-70 °C, pendant 4 à 96 heures, plus préférentiellement 24 à 48 heures.

9. Procédé selon la revendication 7 ou 8, dans lequel la substance de qualité alimentaire pour l'alimentation humaine ou animale consiste en céréales ou en légumes secs/légumineuses, comme l'avoine, le quinoa ou les fèves.

10. Procédé selon l'une quelconque des revendications 7 à 9, dans lequel l'acide aminé est l'alanine, l'histidine, l'isoleucine, la leucine, la lysine, la méthionine, la thréonine, la tyrosine, ou la valine.

11. Procédé selon l'une quelconque des revendications 7 à 10, dans lequel le minéral est le calcium, le fer, ou le zinc.

12. Procédé pour prolonger la durée de conservation d'un produit destiné à l'alimentation humaine ou animale, comprenant la fermentation d'un mélange d'un produit destiné à l'alimentation humaine ou animale ou d'une matière première d'un produit destiné à l'alimentation humaine ou animale et de la souche bactérienne selon la revendication 1, de la culture biologiquement active selon la revendication 2, ou du produit encapsulé selon la revendication 3.

13. Procédé selon la revendication 12, dans lequel
- le mélange comprend la culture biologiquement active selon la revendication 2 à une concentration de 0,01 à 10,00 % vol., rapporté au volume total du mélange, de préférence 0,05 à 5,00 % vol., plus préférentiellement 0,10 à 1,00 % vol., et
- la fermentation du mélange est effectuée à 25-40 °C, de préférence 30-35 °C, pendant 4 à 48 heures, de préférence 12 à 24 heures.

14. Utilisation de la souche selon la revendication 1, de la culture biologiquement active selon la revendication 2, ou du produit encapsulé selon la revendication 3, dans la fabrication d'une composition destinée à l'alimentation humaine ou animale, d'un produit destiné à l'alimentation humaine ou animale, d'une formulation pour l'enrichissement de l'alimentation humaine ou animale, d'un complément dans l'alimentation humaine ou animale, ou d'une formulation nutraceutique.
